# EUROPÄISCHE PATENTSCHRIFT

(11) **EP 0 645 985 B1**
(45) Veröffentlichungstag und Bekanntmachung des Hinweises auf die Patenterteilung: **15.10.1997**
(21) Anmeldenummer: 93909784.6
(22) Anmeldetag: 17.05.1993
(51) Int. Cl.: A61B 17/74

(54) **PLATTEN-SCHRAUBEN-IMPLANTAT**
PLATE-SCREW IMPLANT
IMPLANT A VIS ET A PLAQUES

(30) Priorität: 20.05.1992 DE 4217236
(43) Veröffentlichungstag der Anmeldung: 05.04.1995
(73) Patentinhaber: STUCKENBROCK MEDIZINTECHNIK GmbH, 78532 Tuttlingen (DE)
(72) Erfinder: DITTEL, Karl-Klaus, D-7000 Stuttgart 80 (DE)
(74) Vertreter: Laufhütte, Dieter, Dr.-Ing.
(86) Internationale Anmeldenummer: DE9300445
(87) Internationale Veröffentlichungsnummer: WO9322982

(56) Entgegenhaltungen:
- EP-A- 0 397 059
- WO-A-88/04161
- US-A- 3 256 877
- US-A- 3 554 193

## Beschreibung

Die Erfindung bezieht sich auf ein Platten-Schrauben-Implantat nach dem Oberbegriff des Anspruchs 1.

Derartige Implantate werden in dem Buch "The Dynamic Hip Screw Implant System" von P. Regazzoni, Th. Rüedi, R. Winquist, M. Allgöwer, Springer Verlag beschrieben und mit DHS und DCS bezeichnet. Derartige Implantate finden ihre Anwendung in erster Linie bei Brüchen zwischen Oberschenkelhals und Oberschenkelschaft, also in der pertrochantären Region. Diese Implantate ermöglichen nicht nur eine stabile anatomische Fixierung an der Bruchstelle, sondern auch ein geführtes Zusammenschieben und Zusammendrücken der Bruchflächen durch eine Belastung, weil die an einer Drehung verhinderte Schraube gleitbar geführt ist. Dies hat eine frühzeitige Mobilisierung der Patienten zur Folge, da diese dynamischen Implantate eine Belastung der fixierten Bruchstelle mit vollem Körpergewicht erlauben, was für ältere Patienten, bei denen diese Brüche sehr häufig vorkommen, von Bedeutung ist.

Bei diesen bekannten Implantaten sind Platte und Schenkelrohr einteilig ausgebildet. Da die CCD-Winkel, d.h. die Winkel, die die Schenkelhälse mit den Femurschäften bilden, verschieden sind, müssen Implantate mit unterschiedlichen Winkelstellungen zwischen dem Schenkelrohr und der Platte vorgesehen sein. Allein für den Hüftbereich stehen vier verschiedene Implantate mit Winkeln von 135°, 140°, 145° und 150° zur Verfügung. Zusätzlich müssen verschiedene DCS für entsprechende CCD-Winkelbereiche zur Verfügung gestellt werden. Die Praxis der Applikation von DHS und DCS erfordert eine Vorratshaltung unterschiedlicher Implantate. Da die zur Verfügung stehenden Winkel zwischen dem Schenkelrohr und der Platte in der Praxis um 5° abgestuft sind, kann der Chirurg lediglich eine approximative Anpassung an anatomische Gegebenheiten erzielen.

In vielen Fällen ist ferner eine intraoperative oder nachträgliche Valgisation der Fraktur, insbesondere zur Verhinderung schädlicher Scherbelastungen wünschenswert.

Der Erfindung liegt die Aufgabe zugrunde, ein dynamisches Platten-Schrauben-Implantat, wie vorstehend beschrieben, zur Verfügung zu stellen, durch das die bisherige Typenvielfalt ersetzt wird und das dem Chirurgen eine stufenlose Anpassung an anatomische Gegebenheiten sowie eine intraoperative oder nachträgliche Valgisation ermöglicht.

Erfindungsgemäß wird diese Aufgabe dadurch gelöst, daß zur Einstellung des Winkels zwischen der Platte und dem Schenkelrohr dieses an der Platte um eine parallel sich zur Plattenmittelebene (A-A) erstreckende Achse drehbar gelagert und mittels eines als von außen zugängiges Zahnradgetriebe ausgebildetes Verstellgetriebe um die Achse verschwenkbar und in der eingestellten Winkellage arretierbar ist. Mit besonderem Vorteil ist die drehbare Lagerung derart gestaltet, daß der Winkel zwischen der Platte und dem Schenkelrohr in einem Winkelbereich von 85° bis 155° einstellbar ist.

Zur Versorgung der Patienten mit unterschiedlich gelagerten Brüchen ist es lediglich erforderlich, einen einzigen Platten-Schrauben-Implantattyp zur Verfügung zu haben. Der Chirurg kann dieses Implantat stufenlos an die anatomischen Gegebenheiten des Patienten anpassen. Dadurch, daß die eingestellte Winkellage arretierbar, das heißt festlegbar, ist, werden vom Implantat in sicherer Weise die auftretenden Momente bei Belastung aufgenommen.

Mit Vorteil kann intraoperativ oder nachträglich eine Valgisation, beispielsweise mittels einer Keilosteotomie des Femur durchgeführt werden, da der Winkel zwischen der Platte und dem Schenkelrohr auch beim implantierten Implantat auf den gewünschten Wert eingestellt werden kann.

Zur Arretierung des Schenkelrohrs in der eingestellten Winkellage wird mit Vorteil ein Gehemme oder Gesperre vorgesehen. Mit besonderem Vorteil wird zur Arretierung ein Formgehemme oder Formgesperre verwendet. Dabei ist eines der Gesperreelemente an der Platte und das andere am Schenkelrohr oder an der Drehachse des Schenkelrohres angeordnet.

Diese Verstellung ist insbesondere für die intraoperative oder nachträgliche Valgisation von Vorteil.

Bei einer vorteilhaften Ausführungsform des Erfindungsgegenstandes sind an einem Ende der Platte zwei Gabelschenkel ausgebildet, an denen das Schenkelrohr drehbar gelagert ist. Dabei befindet sich ein Abschnitt des Schenkelrohres zwischen den beiden Gabelschenkeln.

Da sich bei einer DHS das Implantat vom Femur in den unteren Abschnitt des Trochanters hineinerstreckt, sollte eine Anpassung an die Übergangsstelle vom Femur in den Trochanter möglich sein. In vorteilhafter Weise sind zu diesem Zweck die Längsachsen der Gabelschenkel von der Knochenanlageseite der Platte fortgeneigt. Es hat sich als vorteilhaft erwiesen, daß der Neigungswinkel zwischen den Längsachsen dieser Gabelschenkel und der Plattenmittelebene im Bereich von 10° bis 12° liegt.

Bei einer weiteren vorteilhaften Ausführungsform des erfindungsgemäßen Implantats sind in den Gabelschenkeln Lagerzapfen ausgebildet, in denen das Schenkelrohr drehbar gelagert ist.

Ein vorteilhaftes und einfaches Verstellgetriebe zur Einstellung des Winkels zwischen Platte und Schenkelrohr wird dadurch gebildet, daß am zwischen den Gabelschenkeln sich erstreckenden Abschnitt des Schenkelrohrs ein Zahnsegment angeordnet oder ausgebildet ist, welches mit einer Antriebsschnecke kämmt, die im Stegabschnitt der von den Gabelschenkeln gebildeteten Gabel der Platte montiert ist. Dieser vom Zahnsegment und der Antriebsschnecke gebildete Schneckenantrieb ist derart selbsthemmend ausgelegt, daß dieser Schneckenantrieb die normalerweise auftretenden Momente bei einer Belastung des Schenkelrohrs aufnimmt. Mit besonderem Vorteil ist am vom außen zugänglichen Ende der Antriebsschnecke ein Steckschlüsselanschluß ausgebildet. Hierdurch wird die Einstellung des Winkels zwischen Schenkelrohr und Platte vereinfacht.

Um die Montage zu vereinfachen, sind die Lagerzapfen in die Gabelschenkel einsteck- oder einschraubbar. Es ist lediglich erforderlich, ein mit entsprechenden Gegenlagern ausgerüstetes Schenkelrohr zwischen die Gabelenden einzusetzen und dann die Lagerzapfen in ihrer Eingriffslage in den Schenkeln anzubringen.

Ein Ausführungsbeispiel der Erfindung soll unter Bezugnahme auf die Figuren der Zeichnung in der folgenden Beschreibung erläutert werden. Es zeigen:
- Fig. 1: eine schematische Seitenansicht eines als DHS-dynamische Hüftschraube bezeichneten Implantats,
- Fig. 2: eine Ansicht des in Figur 1 dargestellten Implantates von rechts gesehen und
- Fig. 3: eine schematische Schnittansicht des Implantates ohne Schraube.

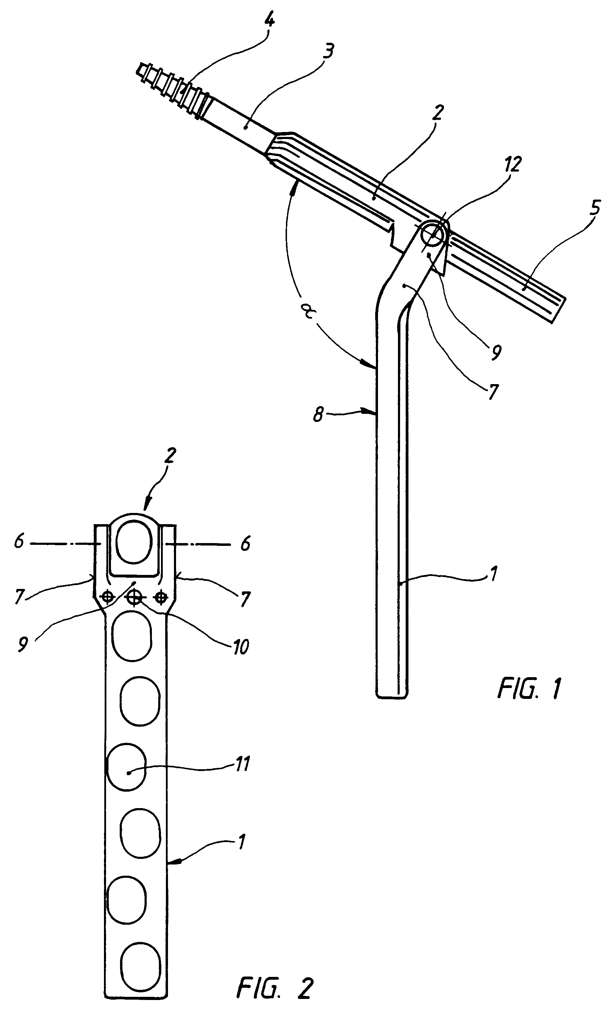

Das in Fig. 1 dargestellte Platten-Schrauben-Implantat (DHS) weist eine Platte 1 auf, die am Femur verschraubt wird, wobei die Schrauben durch die in Figur 2 dargestellten Öffnungen 11 hindurchgeführt werden. Zur Stabilisierung dient die Schraube 3, deren Gewindeabschnitt 4 in den Hüftkopf eingeschraubt wird und deren gewindefreier Schaft 5 formschlüssig und somit unverdrehbar, jedoch axial gleitbar, im Schenkelrohr 2 geführt ist.

Wie insbesondere Figur 2 zeigt, sind an einem Ende der Platte 1 zwei Gabelschenkel 7 ausgebildet. Zwischen diese Gabelschenkel 7 erstreckt sich ein Endabschnitt des Schenkelrohres 2. Dieser Endabschnitt des Schenkelrohres 2 ist zwischen diesen Gabelschenkeln 7 um eine in Figur 2 dargestellte Achse 6 verschwenkbar. Durch diese Verschwenkung kann der in Figur 1 dargestellte Winkel α zwischen dem Schenkelrohr 2 und damit der Schraube 3 einerseits und der Platte 1 andererseits eingestellt werden. In Figur 1 sind schematisch in die Gabelschenkel 7 einschraubbare Drehzapfen 12 dargestellt, die in entsprechende Lagerbohrungen im Schenkelrohr 3 hineinragen.

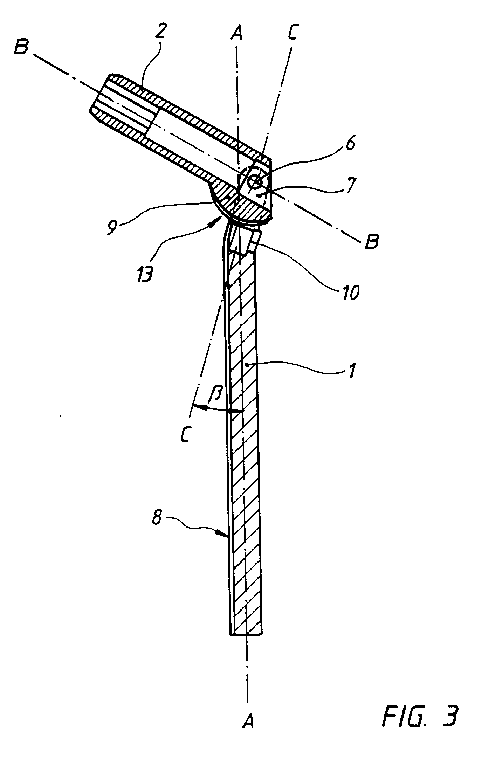

Wie die Figuren 1-3 zeigen, ist am Endabschnitt des Schenkelrohres 3 ein Zahnsegment 9 ausgebildet, dessen Zähne 13 mit einer in der Platte 1 zwischen den Gabelschenkeln 7 angeordneten Schnecke 10 kämmt. Am von außen zugänglichen Ende der Schnecke 10 ist eine Stecköffnung für einen Schlüssel ausgebildet.

Durch eine Betätigung der Schnecke 10 wird der Winkel α zwischen der Mittelebene A-A der Platte 1 und der Achse B-B des Schenkelrohrs 2 auf den erforderlichen Wert eingestellt.

Zur Anpassung des Implantats an die Übergangsstelle zwischen Femur und Trochanter sind die Achsen C-C der Gabelschenkel 7 von der Knochenanlageseite 8 der Platte 1 um den Winkel β fortgeneigt, der im Bereich von 10° bis 12° liegt.

## Patentansprüche

1. Platten-Schrauben-Implantat, an dessen am Femur verschraubbare Platte ein den gewindefreien Schaftabschnitt der Schraube unverdrehbar und gleitbar aufnehmendes Schenkelrohr angeordnet ist, **dadurch gekennzeichnet,** daß zur Einstellung des Winkels (α) zwischen der Platte (1) und dem Schenkelrohr (2) dieses an der Platte (1) um eine parallel sich zur Plattenmittelebene (A-A) erstreckende Achse (6) drehbar gelagert und mittels eines als von außen zugängiges Zahnradgetriebe ausgebildetes Verstellgetriebe um die Achse (6) verschwenkbar und in der eingestellten Winkellage arretierbar ist.

2. Implantat nach Anspruch 1, **dadurch gekennzeichnet,** daß der Winkel (α) zwischen der Platte (1) und dem Schenkelrohr (2) in einem Winkelbereich von 85° - 155° einstellbar ist.

3. Implantat nach Anspruch 1 oder 2, **dadurch gekennzeichnet,** daß zur Arretierung des Schenkelrohres (2) in der eingestellten Winkellage ein Gehemme oder Gesperre vorgesehen ist.

4. Implantat nach mindestens einem der Ansprüche 1-4, **dadurch gekennzeichnet,** daß an einem Ende der Platte (1) zwei Gabelschenkel (7) ausgebildet sind, an denen das Schenkelrohr (2) drehbar gelagert ist.

5. Implantat nach Anspruch 5, **dadurch gekennzeichnet,** daß die Längsachsen (C-C) der Gabelschenkel (7) von der Knochenanlageseite (8) der Platte (1) fortgeneigt sind.

6. Implantat nach Anspruch 6, **dadurch gekennzeichnet,** daß der Neigungswinkel (β) zwischen den Längsachsen (C-C) der Gabelschenkel (7) und der Plattenmittelebene (A-A) im Bereich von 10°-12° liegt.

7. Implantat nach mindestens einem der Ansprüche 5-7, **dadurch gekennzeichnet,** daß in den Gabelschenkeln (7) Lagerzapfen ausgebildet sind, an denen das Schenkelrohr (2) drehbar gelagert ist.

8. Implantat nach mindestens einem der Ansprüche 4-8, **dadurch gekennzeichnet,** daß am zwischen den Gabelschenkeln (7) sich erstreckenden Abschnitt des Schenkelrohres (2) ein Zahnsegment (9) angeordnet oder ausgebildet ist, welches mit einer Antriebsschnecke (10) kämmt, die im Stegabschnitt der von den Gabelschenkeln (7) gebildeten Gabel der Platte (1) montiert ist.

9. Implantat nach Anspruch 9, **dadurch gekennzeichnet,** daß der vom Zahnsegment (9) und der Antriebsschnecke (10) gebildete Schneckenantrieb selbsthemmend ausgelegt ist.

10. Implantat nach Anspruch 9 oder 10, **dadurch gekennzeichnet,** daß am von außen zugänglichen Ende der Antriebsschnecke ein Steckschlüsselanschluß ausgebildet ist.

11. Implantat nach einem der Ansprüche 8-11, **dadurch gekennzeichnet,** daß die Lagerzapfen in die Gabelschenkel (7) einsteck- oder einschraubbar sind.

## Claims

1. Plate-screw implant, of which the plate which is screwed to the femur has arranged upon it an articulated tube element designed to receive and locate so as to prevent rotation the slide-in thread-free shank section of the screw, **characterised in that**, in order to set the angle (α) between the plate (1) and the articulated tube (2), the latter is pivot mounted on the plate (1) to facilitate rotation around a parallel axis (6) extending to the plate centre plane (A-A), may be slewed around the axis (6) by means of an adjusting transmission unit designed as an externally accessible gear assembly, and may be locked in the set angular position.

2. Implant according to Claim 1, **characterised in that** the angle (α) between the plate (1) and the articulated tube (2) is adjustable within a range of 85° - 155°.

3. Implant according to Claim 1 or 2, **characterised in that** a detent or catch is provided for locking the articulated tube (2) in the set angular position.

4. Implant according to at least one of Claims 1 - 3, **characterised in that** two prong-like arms (7) are formed at the end of the plate (1) on which the articulated tube (2) is pivot-mounted.

5. Implant according to Claim 4, **characterised in that** the longitudinal axes (C-C) of the prong-like arms (7) are inclined away from the bone contact face (8) of the plate (1).

6. Implant according to Claim 5, **characterised in that** the angle of inclination (β) between the longitudinal axes (C-C) of the prong-like arms (7) and the plate centre plane (A-A) lies in the region 10° - 12°.

7. Implant according to at least one of Claims 4 - 6 **characterised in that**, formed in the prong-like arms (7) are bearing pins on which the articulated tube (2) is pivot-mounted.

8. Implant according to at least one of Claims 3 - 7, **characterised in that** a serrated segment (9) is arranged or formed on the section of the articulated tube (2) extending between the prong-like arms (7), which serrated segment (9) meshes with a drive wormgear (10) which is mounted in the web section of the fork formed by the prong-like arms (7) of the plate (1).

9. Implant according to Claim 8, **characterised in that** the wormgear drive formed by the serrated segment (9) and the drive worm (10) is of self-locking design.

10. Implant according to Claims 8 or 9, **characterised in that** a socket wrench connection is formed on the externally accessible end of the drive worm.

11. Implant according to one of Claims 7 - 10, **characterised in that** the bearing pins in the prong-like arms (7) are of insertion or screw-in design.

## Revendications

1. Implant à vis et à plaques, sur lequel est disposé sur la plaque pouvant être vissée au fémur un tube articulé recevant la section de la tige de vis sans filet de manière à ce qu'elle ne puisse pas être pivotée et de manière glissante, caractérisé en ce que pour le réglage de l'angle (α) entre la plaque (1) et le tube articulé (2), celui-ci est logé sur la plaque (1) de manière à pouvoir pivoter autour d'un axe (6) s'étendant parallèlement au plan médian de la plaque (A-A) et peut être basculé autour de l'axe (6) au moyen d'un engrenage de réglage formé comme engrenage à roue dentée accessible de l'extérieur et arrêté dans la position angulaire réglée.

2. Implant selon la revendication 1, caractérisé en ce que l'angle (α) entre la plaque (1) et le tube articulé (2) est réglable dans une plage angulaire de 85° à 155°.

3. Implant selon les revendications 1 ou 2, caractérisé en ce qu'un moyen immobilisateur ou de bloquage est prévu pour la position angulaire réglée.

4. Implant selon au moins une des revendications 1 à 3, caractérisé en ce qu'à l'extrémité de la plaque (1) deux branches de fourche (7) sont formées sur lesquelles est monté le tube articulé (2) de manière à pouvoir pivoter.

5. Implant selon la revendication 4, caractérisé en ce que les axes longitudinaux (C-C) des branches de fourche (7) sont inclinées en s'écartant du côté d'appui à l'os (8) de la plaque (1).

6. Implant selon la revendication 5, caractérisé en ce que l'angle d'inclinaison (β) entre les axes longitudinaux (C-C) des branches de fourche (7) et du plan médian de la plaque (A-A) se situe dans la plage de 10° à 12°.

7. Implant selon au moins une des revendications 4 à 6, caractérisé en ce que des tourillons sont formés dans les branches de fourche (7) sur lesquelles est monté le tube articulé (2) de manière à pouvoir pivoter.

8. Implant selon au moins une des revendications 3 à 7, caractérisé en ce qu'à la section du tube articulé (2) s'étendant entre les branches de fourche (7) un segment denté (9) est disposé ou formé qui s'engrène avec une vis sans fin d'engrenage (10) qui est montée dans la partie transversale de la fourchette de la plaque (1) formée par les branches de fourche (7).

9. Implant selon la revendication 8, caractérisé en ce que l'engrenage à vis sans fin formé par le segment denté (9) et la vis sans fin d'engrenage (10) est autobloquant.

10. Implant selon les revendications 8 au 9, caractérisé en ce que sur l'extrémité accessible de l'extérieur de la vis sans fin d'engrenage un raccord pour clé à douille est formé.

11. Implant selon l'une quelconque des revendications 7 à 10, caractérisé en ce que les tourillons peuvent être enfichés ou vissés dans les branches de fourche (7).
